# EUROPEAN PATENT SPECIFICATION

(11) **EP 4 048 337 B1**
(45) Date of publication and mention of the grant of the patent: **19.02.2025**
(21) Application number: 19818249.5
(22) Date of filing: 25.10.2019
(51) Int. Cl.: A61M 1/16, A61M 1/28, A61M 1/36

(54) **MANIFOLD FOR DIALYSIS MACHINES AND EQUIPMENT AND DIALYSIS MACHINE OR EQUIPMENT COMPRISING SAID MANIFOLD**
VERTEILER FÜR DIALYSEMASCHINEN UND GERÄT UND DIALYSEMASCHINE ODER GERÄT MIT DIESEM VERTEILER
COLLECTEUR POUR MACHINES DE DIALYSE ET ÉQUIPEMENT ET MACHINE DE DIALYSE OU ÉQUIPEMENT COMPRENANT LEDIT COLLECTEUR

(43) Date of publication of application: 31.08.2022
(73) Proprietor: IBD Italian Biomedical Devices S.r.l., 46043 Castiglione delle Stiviere (IT)
(72) Inventor: VISOTTI, Andrea, 47121 Forlì (IT); PERAZZINI, Claudia, 46043 Castiglione delle Stiviere (IT); DRUDI, Debora, 46043 Castiglione delle Stiviere (IT)
(74) Representative: Zoli, Filippo
(86) International application number: PCT/IB2019/059172
(87) International publication number: WO 2021/079177

(56) References cited:
- US-A1- 2011 108 474
- US-A1- 2015 165 105
- US-A1- 2017 246 373

## Description

### FIELD OF THE INVENTION

Dialysis treatments, in particular, hemodialysis ones, are widely used in medicine for the extracorporeal removal of harmful substances and/or impurities from the blood such as creatinine and urea, in patients suffering from more or less severe kidney failure.

Unfortunately, kidney failure is a disease widespread on a global scale, in particular in the western world, and in constant growth, even in developing countries and/or geographical areas in which a steady growth is recorded in pathologies attributable to an improved life standard such as diabetes, obesity and the like.

The need to treat an ever-increasing number of subjects suffering from kidney failure has therefore encouraged the development of equipment of different complexity and capacity in accordance with the applications envisaged, in particular, in accordance with whether it is intended for use in hospitals or, on the contrary, on a private basis.

### BACKGROUND ART

In particular, great attention has been paid to the design and/or development of "portable" machines and/or equipment with the dual purpose of streamlining the workload of public organizations on the one hand and, on the other hand, allowing the access to dialysis treatments to a greater number of patients even though with poor and/or limited financial resources.

Further efforts have also been devoted to the development of portable machines and/or equipment adapted to be used in private environments, even though not particularly or poorly equipped, and without the assistance of specialized personnel, as well as being easily connectable to the water and/or electricity network and characterized by low consumption, purchase costs, reduced weight and bulk.

A typical dialysis treatment involves, among other things, the extracorporeal removal of harmful substances and/or impurities from the blood by means of extracorporeal filtering of the blood itself, as well as the removal of excess liquids, such as urine or the like, from the blood, and therefore, ultimately, the extracorporeal circulation of blood and/or other liquids, for example used for adding to the blood substances which it is lacking; since the dialysis treatments represent a practice which is consolidated and known to the skilled in the art, a detailed description is omitted for synthesis purposes.

For the purposes summarized above, such as, for example, the extracorporeal filtering and/or washing of blood or the like, a conventional dialysis machine or equipment must therefore inevitably comprise a plurality of component parts adapted to allow the circulation and/or the extracorporeal circulation of the blood and/or other liquids; by way of non-limiting example, pumps, tanks, filters, valves (two-way or three-way), as well as a plurality of sensors are included in the component parts typically present in a dialysis machine or equipment, such as, for example, flow and/or pressure and/or temperature and/or conductivity sensors, the latter being provided for monitoring the different parameters of both the patient and the equipment.

Furthermore, in an equally obvious manner, the aforesaid component parts must be mutually connected to define, precisely, an extracorporeal circuit, wherein the connection of the aforesaid component parts, according to the most commonly adopted solutions, provides for the use of tubing (for example, flexible silicone tubes), joints and similar connecting components.

The dialysis machines and/or equipment according to the background art briefly described above, although being appreciable according to different points of view, have, however, drawbacks and/or disadvantages that the present invention intends to overcome or at least limit.

A first drawback relates to the fact that, for the realization of the one or more extracorporeal circuits, the use of a not negligible quantity of tubing, usually flexible silicone tubes, is required.

The aforesaid not negligible quantity of tubing does not allow a satisfactory containment of the machine or equipment, making the use thereof, in particular transport, difficult and tiring, especially by the patients themselves.

Furthermore, each tubing section represents a possible risk factor, being the tubing subject to wear and therefore to the formation of leaks, the tubing further being at risk of accidental disconnection.

Therefore, not negligible assembly times are required (with a consequent increase in the relative costs) for the individual connection of each individual tubing, wherein it is also necessary to provide an accurate monitoring of the tubing so as to prevent accidental and unforeseen disconnections and/or the early deterioration thereof with possible leaks.

It is therefore the main object of the present invention to overcome or at least minimize the drawbacks summarized above and observed in the dialysis machines or equipment of the known type.

US 2017/246373 A1, US 2015/165105 A1 and US 2011/108474 A1 disclose disposable of the known type, adapted to be used into dialysis machine and comprising a rigid portion and a flexible part associated to the rigid portion.

In particular, it is the main object of the present invention to provide a dialysis machine or equipment which has a reduced weight and bulk, and which may be assembled by means of simple and fast operations and therefore at low costs.

It is also part of the objects of the present invention to provide a dialysis machine or equipment which provides for a reduced and limited use of flexible pipes and therefore eliminates or at least reduces the risk of accidental disconnections thereof, or in any case the risk of leaks along the one or more circuits for liquids of the machine and/or equipment itself.

It is a further object of the present invention to provide a dialysis machine or equipment which defines one or more circuits for the extracorporeal circulation of liquids, each of said circuits being connectable to circuits and/or equipment exterior to the machine (for example, to the drinking water network) according to equally simple and immediate methods, and so as to ensure maximum reliability of the connections, thus eliminating, even in this case, the risk of accidental disconnections and/or leaks.

In view of the purposes summarized above, the present invention is based on the general consideration according to which the issues or disadvantages observed in machines of the known type may be overcome or at least minimized (thus reaching at the same time the intended objects mentioned above), by replacing, at least for the most part, the flexible tubing of machines or equipment of a known type by means of a manifold which defines therein at least part of the extracorporeal circuits defined as a whole by the machine.

It is in fact apparent that the use of a manifold of the aforesaid type allows reducing the risk of accidental disconnection and/or formation of leaks, as well as connecting the manifold itself to the other components of the machine (pumps, tanks, valves and pipes in general) by means of simple, fast and repeatable methods, and therefore at low costs.

The use of the manifold further allows greatly reducing both the weight and the bulk of the machine or equipment, thus making it easily transportable and usable by the patients themselves and without the assistance of specialized personnel. Furthermore, the use of the manifold allows drastically reducing both management and use costs as well as maintenance costs, the use of the manifold allowing in fact the reduction of maintenance and/or assistance interventions.

Finally, the manifold is suitable to be used also in different types of machines and equipment, thus making it possible to standardize manufacturing and assembly steps.

### DESCRIPTION OF THE PRESENT INVENTION

On the basis of the considerations set forth above and in consideration of the issues and/or drawbacks observed in dialysis machines or equipment of the known type, the present invention relates to a manifold for dialysis machines and/or equipment according to claim 1 and a dialysis machine or equipment according to claim 12. According to an embodiment, the manifold for the circulation of fluids along predetermined circulation paths or circuits, in particular, for extracorporeal dialysis machines, comprises a main body with a plurality of inner channels mutually connected to define said predetermined circulation paths, said predetermined circulation paths communicating with the exterior of said main body by means of at least one inlet and at least one outlet adapted to be connected with component parts and/or tubing of a extracorporeal dialysis machine, said main body comprising a first outer multilayered body, an intermediate multilayered body superimposed on said first outer multilayered body and a second outer multilayered body superimposed on said intermediate multilayered body on the opposite part with respect to said first outer multilayered body, said three bodies being mutually rigidly fastened, wherein the first outer multilayered body has at least a first groove extending both from the surface thereof in contact with the intermediate multilayered body by a depth lower than the whole thickness thereof, as well as perpendicularly to said thickness, wherein said intermediate multilayered body has at least a second groove at said first groove of the first outer multilayered body extending both for the whole thickness of said intermediate multilayered body in a through manner, as well as perpendicularly to said thickness, the two first and second grooves thus defining an inner circulation channel of said fluids, and wherein said second through groove, on a part opposite to said first multilayered body, is closed by said second outer multilayered body.

According to an embodiment, said first outer multilayered body has at least a first through hole extending between the outer surface of said first outer multilayered body opposite to the surface in contact with said intermediate multilayered body and said first groove of said first outer multilayered body, said first through hole thus putting in communication with the exterior a channel or a portion thereof of said plurality of inner channels.

According to an embodiment, said at least a first through hole of said first outer multilayered body defines a seat in which a monitoring component of said circulation of fluids is housed as a pressure and/or temperature and/or conductivity and/or flow sensor, wherein, in said seat the respective monitoring component is housed so as to be in interference with the flow of said fluids in said inner channel or portion thereof.

According to an embodiment, said first outer multilayered body has a plurality of said first through holes positioned each so as to put in communication with the exterior a channel or a portion thereof of said plurality of inner channels, wherein at least two of said first through holes of said first outer multilayered body define each a seat in which a monitoring component of said circulation of fluids is housed as a pressure and/or temperature and/or conductivity and/or flow sensor, and wherein, in each of said at least two seats, the respective monitoring component is housed so as to be in interference with the flow of said liquids.

According to an embodiment, in at least one of said seats, the respective component is housed so as to be accessible from the exterior.

According to an embodiment, at least two of said plurality of first through holes of said first outer multilayered body define an inlet and an outlet, respectively, for the introduction and the discharge of said fluids, respectively, into and from an inner channel or a portion thereof, respectively.

According to an embodiment, said inlet and/or said outlet are shaped so as to be put in fluid communication with component parts of a dialysis machine such as, for example, pumps, and/or filters, and/or tanks, and/or tubing and/or the like. According to an embodiment, a connecting tube is housed in at least said inlet and/or said outlet.

According to an embodiment, said main body has at least a first and a second circulation circuit or path, each connected to the exterior by means of an inlet and an outlet defined by corresponding through holes of said first outer multilayered body.

According to an embodiment, said first and second circulation circuits or paths have a common section.

According to an embodiment, the dialysis machine comprises means adapted to allow the extracorporeal circulation of fluids, such as blood, dialyzing solutions, saline solutions and the like, along pre-determined circulation circuits or paths, said circulation circuits or paths being defined at least partially by a manifold according to one of the embodiments summarized above.

According to an embodiment, said manifold defines at least a first circulation circuit or path whose inlet is connectable to the water mains, wherein at least one pressure sensor and at least one pressure regulator are arranged along said at least a first circulation circuit or path.

According to an embodiment, said at least a first circulation circuit or path is connected to a tank so as to allow the introduction of fluids from said at least a first circuit to said tank and the re-introduction of fluids from said tank into said at least a first circuit.

According to an embodiment, one or more flow and/or conductivity and/or pressure sensors are arranged along said at least a first circulation circuit or path, downstream of said tank.

According to an embodiment, said manifold defines at least a second circulation circuit or path connected to said tank so as to allow the introduction of fluids from said at least a second circuit to said tank and the re-introduction of fluids from said tank into said at least a second circuit.

According to an embodiment, said manifold defines at least one third circulation circuit or path connected both to said tank and to a bicarbonate cartridge, wherein said at least one third circuit is such as to allow the introduction of liquids from said tank to said cartridge and the discharge of liquids from said cartridge.

Any further embodiments of the manifold according to the present invention are defined in the dependent claims.

### BRIEF DESCRIPTION OF THE FIGURES

The present invention will be clarified below by means of the description of some of the embodiments thereof depicted in the attached drawings. It should be noted, however, that the present invention is not limited to the embodiments depicted in the attached drawings; conversely, all those variants or modifications of the embodiments depicted and described below, which will be apparent, obvious and evident to those skilled in the art, are within the field and the scope of the present invention. In particular, in the attached drawings:
Figure 1 shows a diagram of a dialysis machine according to an embodiment of the present invention;
Figures 2a and 2b each show the developments of fluid circulation circuits or paths in the dialysis machine according to an embodiment of the present invention;
Figures 3a and 3b each show the developments of further fluid circulation circuits or paths in the dialysis machine according to an embodiment of the present invention;
Figures 4a and 4b each show the developments of further fluid circulation circuits or paths in the dialysis machine according to an embodiment of the present invention;
Figures 5a, 5b and 5c each show a perspective view of a first multilayered body, an intermediate multilayered body and a second multilayered body, respectively, of a manifold according to an embodiment of the present invention;
Figures 6a and 6b each show a transverse sectional view of a manifold according to an embodiment of the present invention;

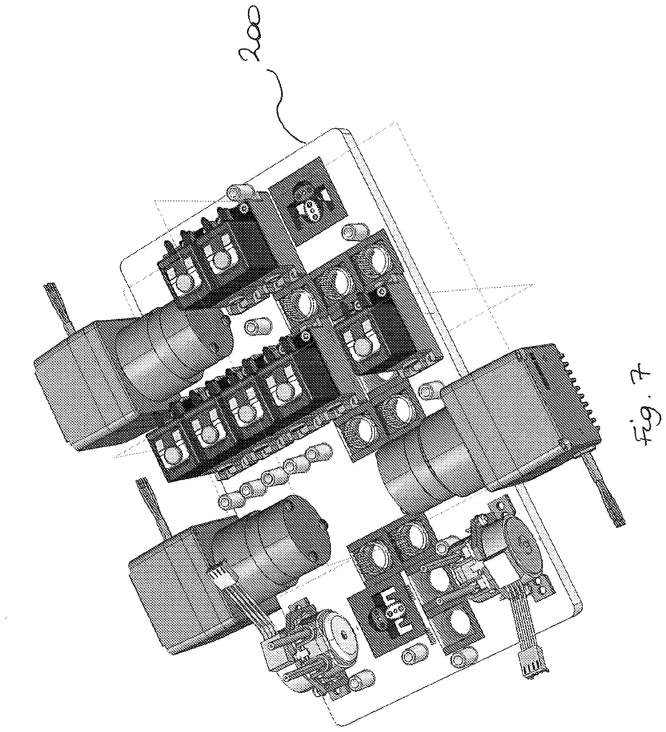

Figure 7 shows a perspective view of a dialysis machine according to an embodiment of the present invention.

### DETAILED DESCRIPTION OF THE PRESENT INVENTION

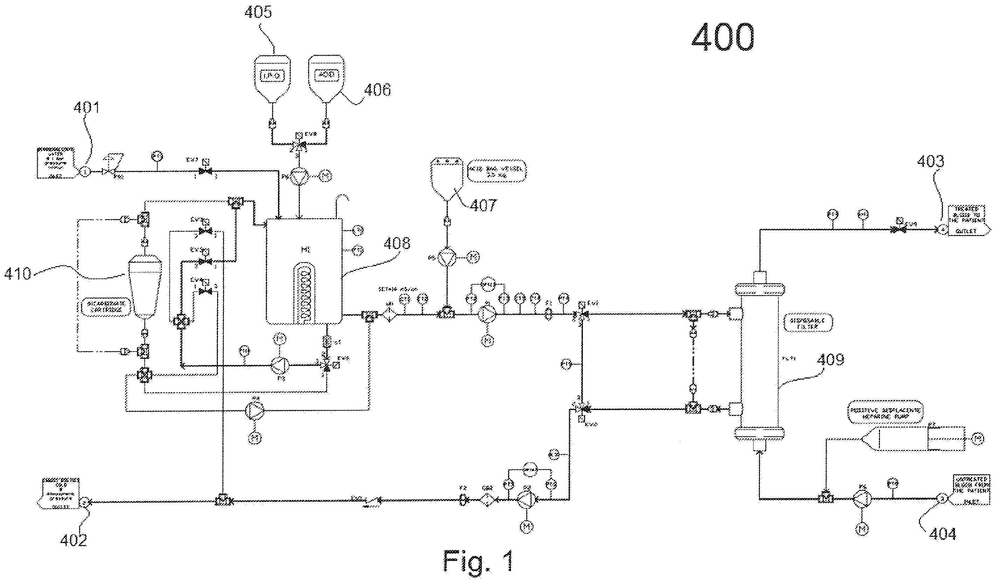

The present invention finds particular and effective application in the case of dialysis machines and/or equipment, this being the reason for which, below, the present invention will be described with particular reference to the application thereof to the case of dialysis machines and/or equipment, the possible applications of the present invention being, however, not limited to the case of dialysis machines or equipment. Figure 1 shows the hydraulic diagram of a dialysis machine according to an embodiment of the present invention.

As shown, the machine 400 comprises an entry or inlet 401 for the introduction of water from the water mains, an outlet 402 for the post-treatment discharge of dialysis liquids, an entry or inlet 404 connectable to the patient for the introduction into the machine 400 of the untreated blood of the patient him or herself, and an outlet 403 connectable to the patient for the post-treatment re-introduction of the treated blood into the body of the patient him or herself.

The machine 400, still as shown, further comprises canonical and conventional components of dialysis machines, such as, for example, tanks 405, 406, 407 and 408 for liquids, a filter 409 and a bicarbonate cartridge 410. The aforesaid canonical components are mutually connected by means of liquid circulation circuits or paths, along which both active components are arranged, such as pumps, valves (one-way and/or two-way and/or three-way) and the like, for moving said liquids, as well as passive components, such as monitoring and/or control components, for example, pressure, temperature, conductivity, flow sensors etc.

Since the symbols used in the diagram of Figure 1 are known to those skilled in the art, since the operation of both the passive and the active components of the machine of Figure 1 is both known to those skilled in the art as well as described briefly below, a detailed description of the aforesaid components is omitted for synthesis purposes. Furthermore, it should be noted that in Figure 1 the hydraulic diagram is shown of a possible embodiment of the dialysis machine according to the present invention, wherein the present invention is not limited to the example of Figure 1, but wherein, on the contrary, according to the present invention, the number and type of active and/or passive components, pumps, valves, tanks etc. may vary according to particular needs and/or circumstances, in particular, according to the specific applications of the machine itself.

As anticipated, in the dialysis machines according to the background art, the components shown in Figure 1, as mentioned, of variable number and type depending on the particular applications, are mutually connected by means of tubing, in particular, very often, by means of flexible silicone tubes.

On the contrary, according to the present invention, the components of the machine 400 are mutually connected by means of circuits or paths for the circulation of liquids (blood, water, solutions etc.) at least in part defined by a manifold according to the following methods.

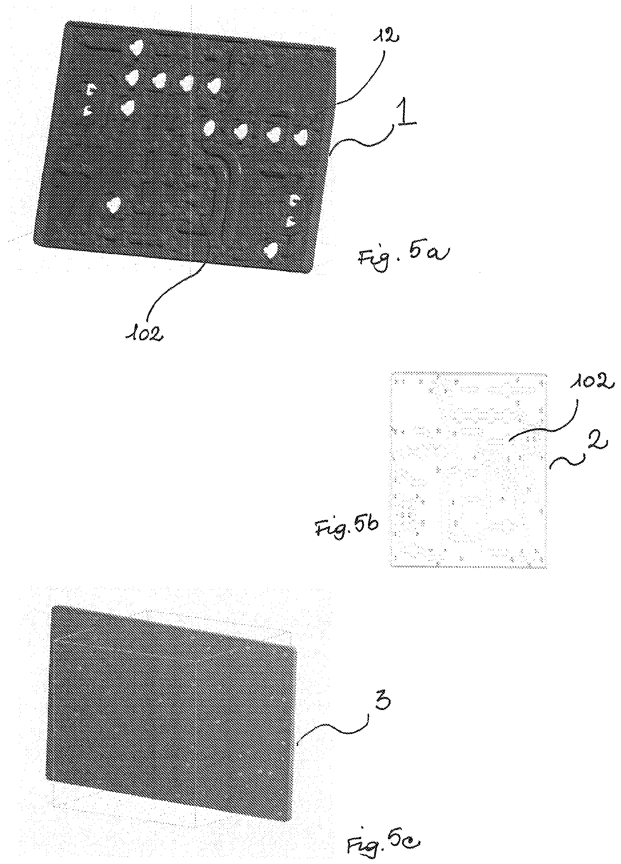

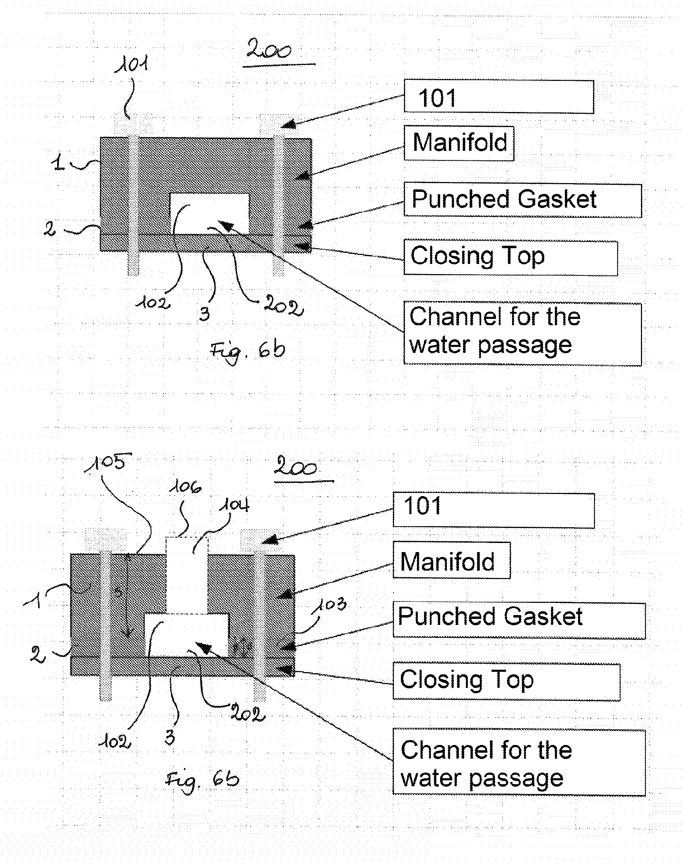

With reference to Figures 5a to 5c, 6a and 6b, a manifold 200 and three multilayered bodies 1, 2 and 3 are shown therein which are mutually superimposed and connected (for example, but not exclusively, by means of screws 101, as shown) to form the manifold 200.

The manifold 200 further has, therein, a plurality of channels 102 mutually connected to define the aforesaid circulation paths or circuits, said channels being in fluid communication with the exterior by means of at least one inlet and/or outlet connected to the component parts of the machine which are not housed in the manifold 200, for example, to one or more of the tanks and/or filters and/or cartridges and/or pump valves of Figure 1, the passive components of the machine (sensors or the like) 400 being on the contrary at least in part housed in the manifold 200 according to the methods explained below.

As shown, the manifold 200 comprises a first outer multilayered body 1, an intermediate multilayered body 2 superimposed on said first outer multilayered body 1 and a second outer multilayered body 3 superimposed on said intermediate multilayered body 2 on a part opposite with respect to said first outer multilayered body 1, said three bodies being mutually rigidly fastened, for example, but not necessarily, by means of screws 101; with regard to the intermediate multilayered body 2, the same may be made of adhesive material to act both as a glue and as a gasket between the outer multilayered bodies 1 and 3. Furthermore, according to alternative embodiments, the intermediate body 2 may be omitted and the bodies 1 and 3 may be mutually fastened by means of different solutions, for example, by means of laser or ultrasound welding.

In particular, the first outer multilayered body 1 has at least one plurality of first grooves or depressions 102 (only one shown in Figure 5a) mutually connected to form one or more channels and/or paths and/or circuits for the circulation of liquids, wherein each first groove 102 extends both from the surface 103 of the outer body 1 in contact with the intermediate multilayered body 2 by a depth lower than the whole thickness S thereof, as well as perpendicularly to said thickness, and therefore perpendicularly to the plane of Figure 6a.

With reference to the intermediate multilayered body 2, the same has a plurality of second grooves 202 each placed at one said first groove 201 of the first outer multilayered body 1, and wherein each second groove 202 extends both for the entire thickness s of said intermediate multilayered body 2 in a passing-through manner, as well as perpendicularly to said thickness s (to the plane of Figure 6a), the first 102 and the second grooves 202 thus defining inner circulation channels of said fluids, wherein each said second through groove (202), from the opposite part to said first multilayered body 1, is closed by said second outer multilayered body 3, also called top.

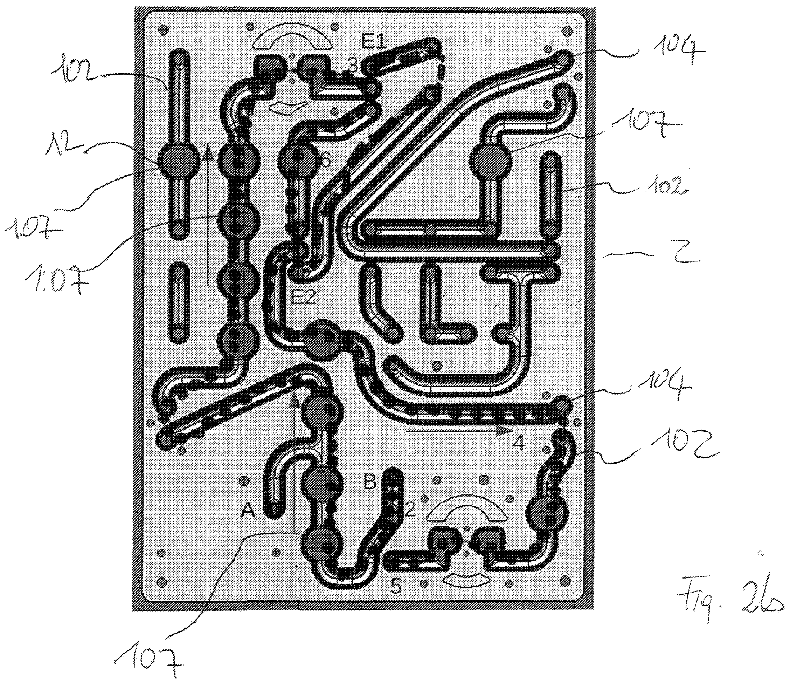

Figure 2b shows a top plan view (from above with respect to Figure 6a) of the first outer multilayered body 1 sectioned at the height of the first grooves 102 (section A-A of Figure 6a) and clearly shows the first grooves 102 connected to form the aforesaid circulation circuits or paths for fluids or liquids (see the following description). Furthermore, the outer layer 1, at said first grooves 102, and along the path thereof, has one or more wells 104 by means of which the first grooves 102 are placed in fluid communication with the exterior of the manifold. Each said well or hole 104 extends, in fact, between the upper surface 105 of the multilayered body 1 and a respective first groove, wherein, therefore, each well or hole 104 may be used as an inlet and respectively as an outlet for the introduction of liquids into the groove 102 (and therefore in the corresponding circulation channel or circuit) and respectively for the discharge of the liquids from the groove 102 (and therefore from the corresponding channel or circuit). The wells or holes 104 are therefore shaped so as to be connected with component parts of a dialysis machine which are exterior to the manifold (valves, pumps, tanks and the like), wherein, for the purpose, according to an embodiment of the present invention, in each well or hole 104 a flexible connecting tube 106, for example made of silicone, is arranged.

Furthermore, with reference to Figure 2b, it is possible to appreciate that the outer multilayered body 1 has a plurality of second wells 107 arranged along the first grooves 102, said second wells 107 each defining a seat for housing respective control and/or monitoring components 12, for example, pressure and/or flow and/or temperature and/or conductivity sensors and/or the like, arranged in the respective housing seats 107 so as to be at least in partial flow interference with the liquid in the underlying groove 102.

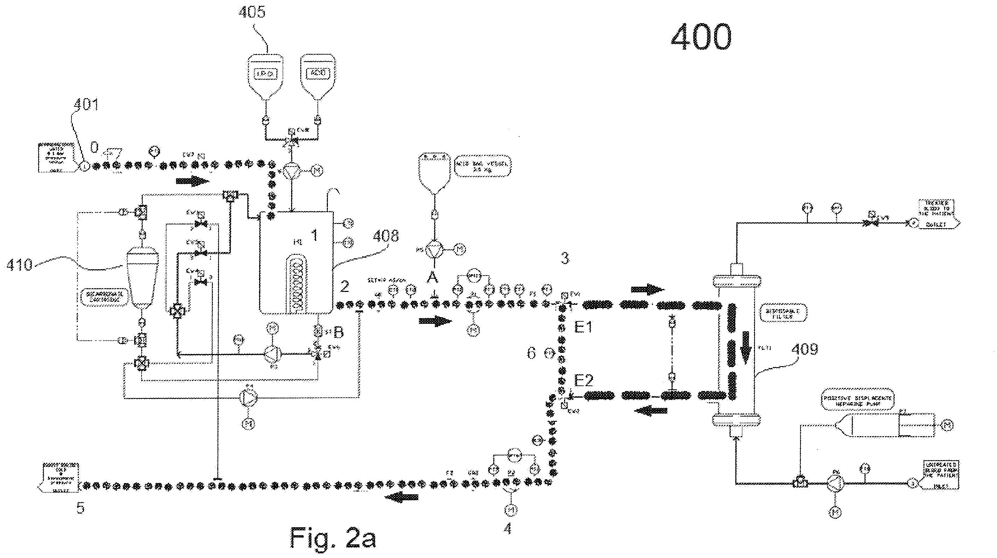

In particular, the second wells 107 may either be dead towards the surface 105 of the multilayered body 1, or open and therefore extend from the respective first groove 102 until surfacing from the surface 105, thereby making the access from the exterior of the manifold to the respective component 12 housed therein possible. With reference to Figures 1, 2a and 2b, a description will be given below of two liquid circulation circuits defined at least in part by a manifold according to an embodiment of the present invention and therefore obtained therein according to the methods clarified above.

As shown, the first circuit comprises two sections or sub-circuits, respectively shown in the Figures by means of a dotted line (first sub-circuit) and a broken line (second sub-circuit).

In the first and second sub-circuits the direction of the flow is that of the arrows.

By means of the first sub-circuit (dotted line) water is drawn, for example, from a hospital network (point 0) and then conveyed along the first section of the sub-circuit, where a pressure sensor, a pressure regulator (Figure 2a), bringing the network pressure from 2-3 bars to 1 bar, and a two-way valve are inserted.

The water is then conveyed to point 1 which is the tank, wherein the water level in the tank 408 is kept constant by means of the upstream two-way valve which opens and closes by virtue of the level sensor inside the tank 408.

From the tank 408 the water is drawn by means of a pump (point 2) and then conveyed along the section downstream of the tank 408 where four conductivity sensors, two control sensors and two protection sensors are present, as well as four pressure sensors and one flow sensor, which are used for control.

This sub-circuit ends at point 3 where there is a deviation valve.

Furthermore, in this first sub-circuit, two injection points are present for the bicarbonate and the acid which are used to concentrate the water (point B and point A).

Finally, the flow continues in point 4, with the suction by means of the second pump, where two pressure sensors and one flow sensor are present, wherein finally the water is discharged (point 5).

The broken line circuit indicates the deviation of the water at point E1, i.e., wherein, once the relative valve is activated, the water is conveyed towards the patient to go to the filter 409 countercurrent and clean the blood.

Once passed through the filter 409 (see arrows) the water is again inserted in the central section (point E2) and then discharged in point 5. When this section or sub-circuit is activated, the segment of point 6 is not crossed by water.

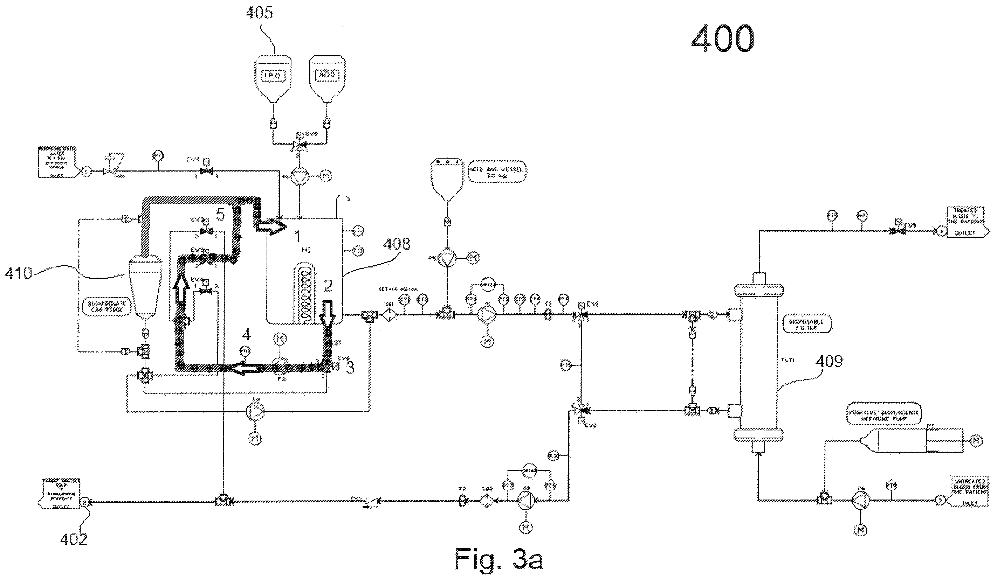

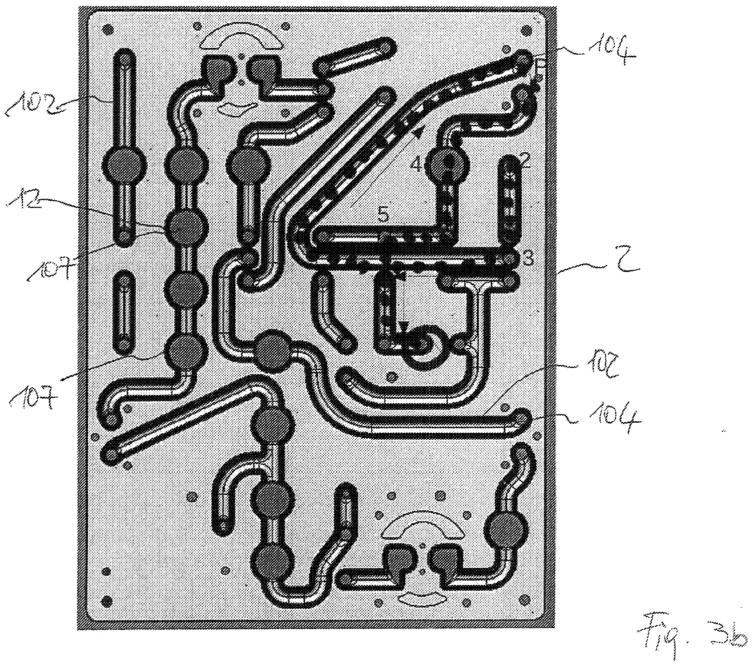

With reference to Figures 1, 3a and 3b, a description will be given below of two further liquid circulation circuits defined at least in part by a manifold according to an embodiment of the present invention and therefore obtained therein according to the methods clarified above.

As shown, also the second circuit consists of two sections or sub-circuits, in particular, a first sub-circuit (dotted line) and a second sub-circuit (continuous line). The circuit indicated with the dotted line is part of the system for degassing and heating the water present inside the tank (point 1).

This circuit, by means of the pump (point P) draws the water in point 2, wherein the water, by means of the pawl and a three-way valve (calibrated restriction of 1 mm in diameter, point 3, with a formation of a local depression which facilitates the escape of air) is conveyed through a pressure sensor (point 4, used for checking the correct flow) and, by means of a solenoid valve (point 5), is reintroduced into the tank 408; in this case it is therefore a continuous cycle (see arrows direction).

The flow set to have an excellent degassing is of about 2 I/min.

Inside the tank 408 there is a heating cartridge which keeps the liquid at 37 degrees. The circuit highlighted with a continuous line allows maintaining a constant level inside the bicarbonate cartridge 410 during the treatment, wherein the liquid flows due to load losses.

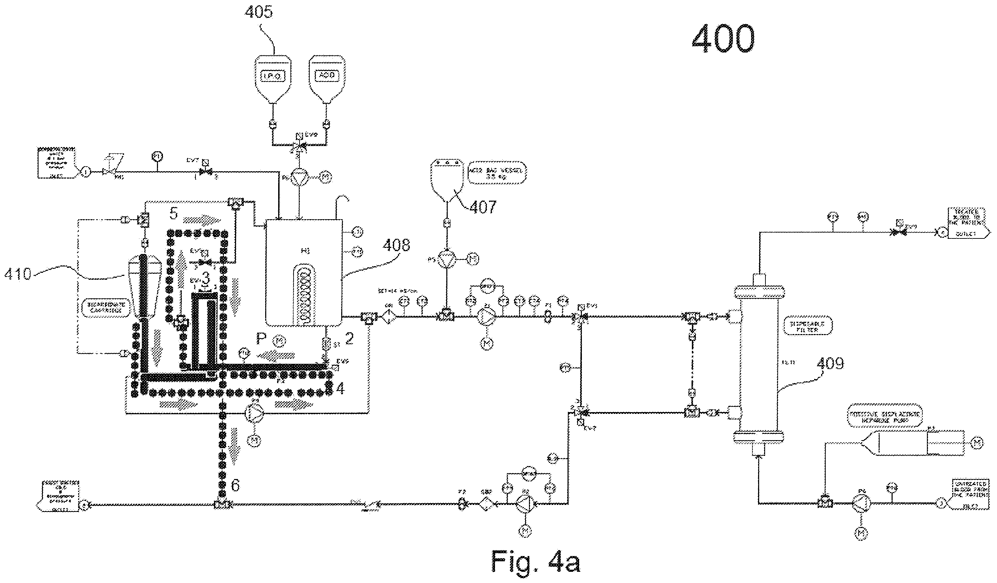

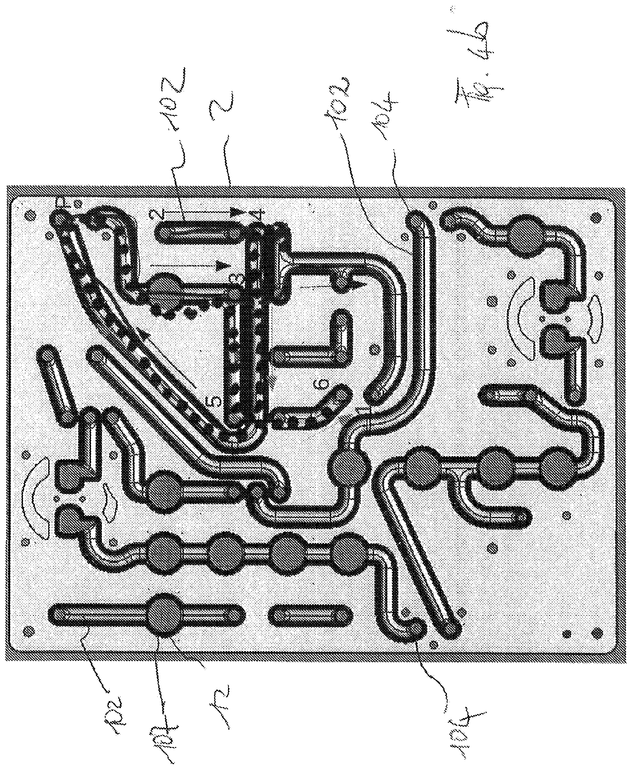

With reference to Figures 1, 4a and 4b, a description will be given below of a further liquid circulation circuit defined at least in part by a manifold according to an embodiment of the present invention and therefore obtained therein according to the methods clarified above.

The circuit is composed of two sections, one shown by means of a dotted line and the other by means of a continuous line, which intervene in two distinct steps of the operation of the machine.

The continuous line circuit serves to hydrate the bicarbonate cartridge 410 (point 1) and to create a supersaturated solution which is then diluted. Hydration consists in drawing water (point 2) from the tank 408, by using the pump P (the same as the one of the degassing circuit), wherein water is introduced into the bicarbonate cartridge 410 by means of the valve (point 3).

In the case of the circuit section represented by the continuous line, this has the function of emptying the cartridge 410 at the end of the treatment. The suction of the pump (point p) is deviated by the valve (point 4), which draws the water from the inside of the cartridge 410 (point 1), wherein the water drawn is conveyed to the outlet (point 6) through the valve (point 5).

It has thus been shown, by means of the above detailed description of the embodiments of the present invention depicted in the drawings, that the present invention allows achieving the preset aims and/or to overcome or at least minimize the typical drawbacks of the solutions according to the background art.

In particular, the present invention allows the making of a dialysis machine or equipment which provides for a reduced and limited use of flexible pipes and therefore eliminates or at least reduces the risk of accidental disconnections thereof, or in any case the risk of leaks along the one or more circuits for liquids of the machine and/or equipment itself.

Furthermore, the present invention provides a dialysis machine or equipment which defines one or more circuits for the extracorporeal circulation of liquids, each of said circuits being connectable to circuits and/or equipment exterior to the machine (for example, to the drinking water network) according to equally simple and immediate methods, and so as to ensure maximum reliability of the connections, thus eliminating, even in this case, the risk of accidental disconnections and/or leaks. Finally, the machine or equipment according to the present invention requires minimal, simple and inexpensive maintenance, ensures constant reliability and a high level of performance over a sufficiently long period of time, as well as the maximum level of hygiene and the minimum risk of infections and/or contamination. Although the present invention has been clarified by means of the detailed description of the embodiments thereof depicted in the drawings, the present invention is obviously not limited to the embodiments described above and depicted in the drawings; conversely, all those variants of the embodiments described and depicted, which will be obvious and evident to those skilled in the art, are within the scope of the present invention.

The object of the present invention is thus defined by the claims.

## Claims

1. A manifold (200) for the circulation of fluids along predetermined circulation paths or circuits, in particular for extracorporeal dialysis machines (100), said manifold (200) comprising a main body with a plurality of inner channels mutually connected so as to define said predetermined circulation paths, said predetermined circulation paths communicating with the exterior of said main body by means of at least one inlet and at least one outlet adapted to be connected to component parts and/or tubing of an extracorporeal dialysis machine, **characterized in that** said main body comprises a first outer multilayered body (1) and a second outer multilayered body (3) superimposed on said first outer multilayered body (1), said two bodies being mutually and rigidly fastened, and **in that** the first outer multilayered body (1) has at least a first groove (102) extending both from the surface (103) thereof in contact with the second outer multilayered body (3), over a depth lower than the whole thickness (S) thereof, and perpendicularly to said thickness (S), and **in that** said first through-groove (102), on its side opposite to said first multilayered body (1) is closed by said second outer multilayered body (3),
by the fact that said first outer multilayered body (1) has at least a first through hole (104, 107) extending between the outer surface (105) of said first outer multilayered body (1) opposite to the surface (103) in contact with said second outer multilayered body (3) and said first groove (102) of said first outer multilayered body (1), said first through hole (104, 107) thus putting in communication with the exterior a channel or a portion thereof of said plurality of inner channels and
by the fact that said at least a first through hole (107) of said first outer multilayered body (1) defines a seat wherein a monitoring component (12) of said circulation of fluids, such as a pressure and/or temperature and/or conductivity and/or flow sensor, is housed, and **in that** the respective monitoring component (12) is housed in said seat (11) so as to be in interference with the flow of said fluids in said inner channel or portion thereof.

2. A manifold according to claim 1, **characterized in that** said first outer multilayered body (1) has a plurality of said first through holes (104, 107) each positioned so as to put in communication with the exterior a channel or a portion thereof of said plurality of inner channels, and **in that** at least two of said first through holes (104, 107) of said first outer multilayered body (1) define each a seat (107) wherein a monitoring component (12) of said circulation of fluids is housed, such as a pressure and/or temperature and/or conductivity and/or flow sensor, and **in that** in each of said at least two seats (107) the respective monitoring component (12) is housed so as to be in interference with the flow of said liquids.

3. A manifold (200) according to claim 1 or 2, **characterized in that** in at least one of said seats (107) the respective component (12) is housed so as to be accessible from the exterior.

4. A manifold according to one of claims 2 to 3, **characterized in that** at least two of said plurality of first through holes (104) of said first outer multilayered body (1) define an inlet and an outlet, respectively, for the introduction and the discharge of said fluids, respectively, into and from an inner channel or a portion thereof, respectively.

5. A manifold according to claim 4, **characterized in that** said inlet and/or said outlet are shaped so as to be put in fluid communication with component parts of a dialysis machine such as, for example, pumps, and/or filters, and/or tanks, and/or tubing and/or the like.

6. A manifold (200) according to claim 5, **characterized in that** a connecting tube (106) is housed in at least said inlet and/or said outlet.

7. A manifold (200) according to one of claims 1 to 6, **characterized in that** said main body has at least a first and a second circulation circuit or path, each connected to the exterior by means of an inlet and an outlet defined by corresponding through holes (104) of said first outer multilayered body (1)

8. A manifold according to claim 7, **characterized in that** said first and second circulation circuits or paths have a common section.

9. A manifold (200) according to one of claims 1 to 8, **characterized in that** said main body comprises an intermediate multilayered body (2) interposed between said first (1) and second outer multilayered bodies (3) and **in that** said intermediate multilayered body (2) has at least a second groove (202) in correspondence of said first groove (102) of the first outer multilayered body (1) extending both over the whole thickness of said intermediate multilayered body (2) in a passing-through manner, and perpendicularly to said thickness, the two first (102) and second grooves (202) therefore defining an inner channel for the circulation of said fluids.

10. A dialysis machine comprising means adapted to allow the extracorporeal circulation of fluids, such as blood, dialyzing solutions, saline solutions and the like, along pre-determined circulation circuits or paths, **characterized in that** said circulation circuits or paths are defined at least partially by a manifold according to one of claims 1 to 9.

11. The dialysis machine according to claim 10, **characterized in that** said manifold defines at least a first circulation circuit or path whose inlet is connectable to the water mains, and **in that** at least one pressure sensor and at least one pressure regulator are arranged along said at least a first circulation circuit or path.

12. The dialysis machine according to one of claims 10 and 11, **characterized in that** said at least a first circulation circuit or path is connected to a tank so as to allow the introduction of fluids from said at least a first circuit to said tank and the re-introduction of fluids from said tank into said at least a first circuit.

13. The dialysis machine according to claim 12, **characterized in that** one or more flow and/or conductivity and/or pressure sensors are arranged along said at least a first circulation circuit or path, downstream of said tank.

14. The dialysis machine according to one of claims 10 to 13, **characterized in that** said manifold defines at least a second circulation circuit or path connected to said tank so as to allow the introduction of fluids from said at least a second circuit to said tank and the re-introduction of fluids from said tank into said at least a second circuit.

15. The dialysis machine according to one of claims 12 to 14, **characterized in that** said manifold defines at least one third circulation circuit or path connected both to said tank and to a bicarbonate cartridge, and **in that** said at least one third circuit is such as to allow the introduction of liquids from said tank to said cartridge and the discharge of liquids from said cartridge.

## Patentansprüche

1. Verteiler (200) für die Kreislaufführung von Fluiden entlang vorbestimmter Umlaufwege oder -kreisläufe, insbesondere für extrakorporale Dialysegeräte (100), wobei der Verteiler (200) einen Hauptkörper mit einer Vielzahl von inneren Kanälen umfasst, die wechselseitig miteinander verbunden sind, um die vorbestimmten Umlaufwege definieren, wobei die vorbestimmten Umlaufwege mit der Umgebung des Hauptkörpers über mindestens einen Einlass und mindestens einen Auslass in Verbindung stehen, die dazu ausgebildet sind, mit Bauteilen und/oder Verrohrungen eines extrakorporalen Dialysegeräts verbunden zu werden,
**dadurch gekennzeichnet, dass** der Hauptkörper einen ersten äußeren mehrschichtigen Körper (1) und einen zweiten äußeren mehrschichtigen Körper (3) umfasst, der auf dem ersten äußeren mehrschichtigen Körper (1) aufliegt, wobei die beiden Körper wechselseitig und starr befestigt sind, und dass der erste äußere mehrschichtige Körper (1) mindestens eine erste Nut (102) aufweist, die sich sowohl von dessen Oberfläche (103) in Kontakt mit dem zweiten äußeren mehrschichtigen Körper (3) über eine geringere Tiefe als dessen gesamte Dicke (S) als auch senkrecht zu dieser Dicke (S) erstreckt,
und dass die erste durchgehende Nut (102) auf ihrer dem ersten mehrschichtigen Körper (1) gegenüberliegenden Seite durch den zweiten äußeren mehrschichtigen Körper (3) verschlossen ist, dadurch, dass der erste äußere mehrschichtige Körper (1) mindestens ein erstes durchgehendes Loch (104, 107) aufweist, das sich zwischen der äußeren Oberfläche (105) des ersten äußeren mehrschichtigen Körpers (1) gegenüber der Oberfläche (103) in Kontakt mit dem zweiten äußeren mehrschichtigen Körper (3) und der ersten Nut (102) des ersten äußeren mehrschichtigen Körpers (1) erstreckt, wodurch das erste durchgehende Loch (104, 107) einen Kanal oder einen Teil davon aus der Mehrzahl innerer Kanäle mit der Umgebung verbindet,
und dadurch, dass mindestens ein erstes durchgehendes Loch (107) des ersten äußeren mehrschichtigen Körpers (1) einen Sitz definiert, in dem eine Überwachungskomponente (12) der Kreislaufführung von Fluiden, wie beispielsweise ein Druck- und/oder Temperatur- und/oder Leitfähigkeits- und/oder Durchflusssensor, aufgenommen ist, und dass die jeweilige Überwachungskomponente (12) in dem Sitz (11) so aufgenommen ist, dass sie mit dem Fluss der Fluide in dem inneren Kanal oder einem Teil davon in Wechselwirkung steht.

2. Verteiler nach Anspruch 1, **dadurch gekennzeichnet, dass** der erste äußere mehrschichtige Körper (1) eine Mehrzahl der ersten durchgehenden Löcher (104, 107) aufweist, die jeweils so positioniert sind, dass sie einen Kanal oder einen Teil davon aus der Mehrzahl der inneren Kanäle mit der Umgebung verbinden, und dass mindestens zwei der ersten durchgehenden Löcher (104, 107) des ersten äußeren mehrschichtigen Körpers (1) jeweils einen Sitz (107) definieren, in dem eine Überwachungskomponente (12) der Kreislaufführung von Fluiden untergebracht ist, wie beispielsweise ein Druck- und/oder Temperatur- und/oder Leitfähigkeits- und/oder Durchflusssensor, und dass in jedem der mindestens zwei Sitze (107) die jeweilige Überwachungskomponente (12) so aufgenommen ist, dass sie mit dem Fluss der Fluiden in Wechselwirkung steht.

3. Verteiler (200) nach Anspruch 1 oder 2, **dadurch gekennzeichnet, dass** in mindestens einem der Sitze (107) die jeweilige Komponente (12) so aufgenommen ist, dass sie von der Umgebung aus zugänglich ist.

4. Verteiler gemäß einem der Ansprüche 2 bis 3, **dadurch gekennzeichnet, dass** mindestens zwei der Mehrzahl erster durchgehender Löcher (104) des ersten äußeren mehrschichtigen Körpers (1) einen Einlass bzw. einen Auslass für die Zufuhr bzw. die Ableitung der Fluide in einen bzw. aus einem inneren Kanal oder einem Teil davon definieren.

5. Verteiler nach Anspruch 4, **dadurch gekennzeichnet, dass** der Einlass und/oder der Auslass so geformt sind, dass sie mit Bauteilen eines Dialysegeräts, wie beispielsweise Pumpen und/oder Filtern und/oder Tanks und/oder Verrohrungen und/oder dergleichen, in Fluidverbindung gebracht werden können.

6. Verteiler (200) nach Anspruch 5, **dadurch gekennzeichnet, dass** ein Verbindungsrohr (106) in mindestens dem Einlass und/oder dem Auslass aufgenommen ist.

7. Verteiler (200) nach einem der Ansprüche 1 bis 6, **dadurch gekennzeichnet, dass** der Hauptkörper mindestens einen ersten und einen zweiten Umlaufweg oder -kreislauf aufweist, die jeweils über einen Einlass und einen Auslass, die durch entsprechende durchgehende Löcher (104) des ersten äußeren mehrschichtigen Körpers (1) definiert sind, mit der Umgebung verbunden sind.

8. Verteiler gemäß Anspruch 7, **dadurch gekennzeichnet, dass** der erste und der zweite Umlaufkreislauf oder -weg einen gemeinsamen Abschnitt aufweisen.

9. Verteiler (200) gemäß einem der Ansprüche 1 bis 8, **dadurch gekennzeichnet, dass** der Hauptkörper einen mehrschichtigen Zwischenkörper (2) umfasst, der zwischen dem ersten (1) und dem zweiten äußeren mehrschichtigen Körper (3) angeordnet ist, und dass der mehrschichtige Zwischenkörper (2) mindestens eine zweite Nut (202) in Übereinstimmung mit der ersten Nut (102) des ersten äußeren mehrschichtigen Körpers (1) aufweist, die sich sowohl über die gesamte Dicke des mehrschichtigen Zwischenkörpers (2) in einer durchgehenden Weise als auch senkrecht zu dieser Dicke erstreckt, wobei die beiden ersten (102) und zweiten Nuten (202) daher einen inneren Kanal für die Kreislaufführung von Fluiden definieren.

10. Dialysegerät mit Mitteln, die dazu ausgebildet sind, die extrakorporale Kreislaufführung von Fluiden, wie Blut, Dialyselösungen, Salzlösungen und dergleichen, entlang vorbestimmter Umlaufkreise oder -wege zu ermöglichen, **dadurch gekennzeichnet, dass** die Umlaufkreise oder -wege zumindest teilweise durch einen Verteiler gemäß einem der Ansprüche 1 bis 9 definiert sind.

11. Dialysegerät nach Anspruch 10, **dadurch gekennzeichnet, dass** der Verteiler mindestens einen ersten Umlaufkreislauf oder -weg definiert, dessen Einlass mit der Wasserleitung verbunden werden kann, und dass mindestens ein Drucksensor und mindestens ein Druckregler entlang des mindestens ersten Umlaufkreislaufs oder -wegs angeordnet sind.

12. Dialysegerät gemäß einem der Ansprüche 10 und 11, **dadurch gekennzeichnet, dass** der mindestens eine erste Umlaufweg oder -kreislauf mit einem Tank verbunden ist, um die Zufuhr von Fluiden aus dem mindestens einen ersten Kreislauf in den Tank und die erneute Zufuhr von Fluiden aus dem Tank in den mindestens einen ersten Kreislauf zu ermöglichen.

13. Dialysegerät nach Anspruch 12, **dadurch gekennzeichnet, dass** ein oder mehrere Durchfluss- und/oder Leitfähigkeits- und/oder Drucksensoren entlang des mindestens ersten Umlaufwegs oder -kreislaufs stromabwärts des Tanks angeordnet sind.

14. Dialysegerät gemäß einem der Ansprüche 10 bis 13, **dadurch gekennzeichnet, dass** der Verteiler mindestens einen zweiten Umlaufweg oder -kreislauf definiert, der mit dem Tank verbunden ist, um die Zufuhr von Fluiden aus dem mindestens einen zweiten Kreislauf in den Tank und die erneute Zufuhr von Fluiden aus dem Tank in den mindestens einen zweiten Kreislauf zu ermöglichen.

15. Dialysegerät gemäß einem der Ansprüche 12 bis 14, **dadurch gekennzeichnet, dass** der Verteiler mindestens einen dritten Umlaufweg oder -kreislauf definiert, der sowohl mit dem Tank als auch mit einer Bicarbonatkartusche verbunden ist, und dass der mindestens eine dritte Kreislauf so beschaffen ist, dass er die Zufuhr von Flüssigkeiten aus dem Tank zu der Kartusche und die Ableitung von Flüssigkeiten aus der Kartusche ermöglicht.

## Revendications

1. - Collecteur (200) pour la circulation de fluides le long de trajets ou de circuits de circulation prédéterminés, notamment pour des machines de dialyse extracorporelle (100), ledit collecteur (200) comprenant un corps principal avec une pluralité de canaux internes mutuellement reliés de manière à définir lesdits trajets de circulation prédéterminés, lesdits trajets de circulation prédéterminés communiquant avec l'extérieur dudit corps principal au moyen d'au moins une entrée et d'au moins une sortie aptes à être reliées à des parties de composant et/ou à un tube d'une machine de dialyse extracorporelle, **caractérisé par le fait que** ledit corps principal comprend un premier corps multicouche externe (1) et un deuxième corps multicouche externe (3) superposé audit premier corps multicouche externe (1), lesdits deux corps étant mutuellement et rigidement fixés, et **par le fait que** le premier corps multicouche externe (1) a au moins une première rainure (102) s'étendant à la fois depuis sa surface (103) en contact avec le deuxième corps multicouche externe (3), sur une profondeur inférieure à toute son épaisseur (S), et perpendiculairement à ladite épaisseur (S), et **par le fait que** ladite première rainure traversante (102), sur son côté opposé audit premier corps multicouche (1), est fermée par ledit deuxième corps multicouche externe (3),
**par le fait que** ledit premier corps multicouche externe (1) a au moins un premier trou traversant (104, 107) s'étendant entre la surface extérieure (105) dudit premier corps multicouche externe (1) opposée à la surface (103) en contact avec ledit second corps multicouche externe (3) et ladite première rainure (102) dudit premier corps multicouche externe (1), ledit premier trou traversant (104, 107) mettant ainsi en communication avec l'extérieur un canal, ou une partie de celui-ci, de ladite pluralité de canaux internes, et
**par le fait que** ledit au moins un premier trou traversant (107) dudit premier corps multicouche externe (1) définit un siège dans lequel est reçu un composant de surveillance (12) de ladite circulation de fluides, tel qu'un capteur de pression et/ou de température et/ou de conductivité et/ou de débit, et que le composant de surveillance respectif (12) est reçu dans ledit siège (11) de manière à être en interférence avec l'écoulement desdits fluides dans ledit canal interne ou ladite partie de celui-ci.

2. - Collecteur selon la revendication 1, **caractérisé par le fait que** ledit premier corps multicouche externe (1) a une pluralité desdits premiers trous traversants (104, 107) positionnés chacun de manière à mettre en communication avec l'extérieur un canal, ou une partie de celui-ci, de ladite pluralité de canaux internes, et **par le fait qu'**au moins deux desdits premiers trous traversants (104, 107) dudit premier corps multicouche externe (1) définissent chacun un siège (107) dans lequel est reçu un composant de surveillance (12) de ladite circulation de fluides, tel qu'un capteur de pression et/ou de température et/ou de conductivité et/ou de débit, et que dans chacun desdits au moins deux sièges (107) le composant de surveillance (12) respectif est reçu de manière à être en interférence avec l'écoulement desdits liquides.

3. - Collecteur (200) selon la revendication 1 ou 2, **caractérisé par le fait que** dans au moins un desdits sièges (107) le composant respectif (12) est reçu de manière à être accessible depuis l'extérieur.

4. - Collecteur selon l'une des revendications 2 à 3, **caractérisé par le fait qu'**au moins deux de ladite pluralité de premiers trous traversants (104) dudit premier corps multicouche externe (1) définissent une entrée et une sortie, respectivement, pour l'introduction et l'évacuation desdits fluides, respectivement, dans et depuis un canal interne ou une partie de celui-ci, respectivement.

5. - Collecteur selon la revendication 4, **caractérisé par le fait que** ladite entrée et/ou ladite sortie sont conformées de manière à être mises en communication fluidique avec des éléments constitutifs d'une machine de dialyse tels que, par exemple, des pompes, et/ou des filtres, et/ou des réservoirs, et/ou des tubes et/ou analogues.

6. - Collecteur (200) selon la revendication 5, **caractérisé par le fait qu'**un tube de raccordement (106) est reçu dans au moins ladite entrée et/ou ladite sortie.

7. - Collecteur (200) selon l'une des revendications 1 à 6, **caractérisé par le fait que** ledit corps principal a au moins un premier et un deuxième circuit ou trajet de circulation, chacun relié à l'extérieur au moyen d'une entrée et d'une sortie définies par des trous traversants correspondants (104) dudit premier corps multicouche externe (1).

8. - Collecteur selon la revendication 7, **caractérisé par le fait que** lesdits premier et deuxième circuits ou trajets de circulation ont une section commune.

9. - Collecteur (200) selon l'une des revendications 1 à 8, **caractérisé par le fait que** ledit corps principal comprend un corps multicouche intermédiaire (2) interposé entre lesdits premier (1) et second corps (3) multicouches externes et **par le fait que** ledit corps multicouche intermédiaire (2) a au moins une deuxième rainure (202) en correspondance de ladite première rainure (102) du premier corps multicouche externe (1) s'étendant sur toute l'épaisseur dudit corps multicouche intermédiaire (2) de manière traversante, et perpendiculairement à ladite épaisseur, les deux première (102) et deuxième (202) rainures définissant ainsi un canal interne pour la circulation desdits fluides.

10. - Machine de dialyse comprenant des moyens agencés pour permettre la circulation extracorporelle de fluides, tels que du sang, des solutions de dialyse, des solutions salines et analogues, le long de circuits ou trajets de circulation prédéterminés, **caractérisée par le fait que** lesdits circuits ou trajets de circulation sont définis au moins partiellement par un collecteur selon l'une des revendications 1 à 9.

11. - Machine de dialyse selon la revendication 10, **caractérisée par le fait que** ledit collecteur définit au moins un premier circuit ou trajet de circulation dont l'entrée est apte à être reliée au réseau d'eau, et **par le fait qu'**au moins un capteur de pression et au moins un régulateur de pression sont disposés le long dudit au moins un premier circuit ou trajet de circulation.

12. - Machine de dialyse selon l'une des revendications 10 et 11, **caractérisée par le fait que** ledit au moins un premier circuit ou trajet de circulation est relié à un réservoir de manière à permettre l'introduction de fluides dudit au moins un premier circuit vers ledit réservoir et la réintroduction de fluides dudit réservoir dans ledit au moins un premier circuit.

13. - Machine de dialyse selon la revendication 12, **caractérisée par le fait qu'**un ou plusieurs capteurs de débit et/ou de conductivité et/ou de pression sont disposés le long dudit au moins un premier circuit ou trajet de circulation, en aval dudit réservoir.

14. - Machine de dialyse selon l'une des revendications 10 à 13, **caractérisée par le fait que** ledit collecteur définit au moins un deuxième circuit ou trajet de circulation relié audit réservoir de manière à permettre l'introduction de fluides dudit au moins un deuxième circuit vers ledit réservoir et la réintroduction de fluides dudit réservoir dans ledit au moins un deuxième circuit.

15. - Machine de dialyse selon l'une des revendications 12 à 14, **caractérisée par le fait que** ledit collecteur définit au moins un troisième circuit ou trajet de circulation relié à la fois audit réservoir et à une cartouche de bicarbonate, et **par le fait que** ledit au moins un troisième circuit est tel qu'il permet l'introduction de liquides dudit réservoir vers ladite cartouche et l'évacuation de liquides à partir de ladite cartouche.
